# EUROPEAN PATENT SPECIFICATION

(11) **EP 3 327 156 B1**
(45) Date of publication and mention of the grant of the patent: **11.10.2023**
(21) Application number: 16840729.4
(22) Date of filing: 16.08.2016
(51) Int. Cl.: C22C 1/08, B22F 3/11, C22C 1/04, C22C 14/00, C22C 19/07, C22C 27/02, C22C 38/44

(54) **POROUS METAL MATERIAL AND PREPARATION METHOD THEREFOR**
PORÖSES METALLMATERIAL UND HERSTELLUNGSVERFAHREN DAFÜR
MATÉRIAU MÉTALLIQUE POREUX ET PROCÉDÉ DE PRÉPARATION ASSOCIÉ

(30) Priority: 31.08.2015 CN 201510543058
(43) Date of publication of application: 30.05.2018
(73) Proprietor: Chongqing Runze Pharmaceutical Co. Ltd., Chongqing 400042 (CN)
(72) Inventor: YE, Lei, Chongqing 400042 (CN)
(74) Representative: Eisenführ Speiser
(86) International application number: PCT/CN2016/095419
(87) International publication number: WO 2017/036300

(56) References cited:
- WO-A1-2013/086504
- CN-A- 101 418 391
- CN-A- 101 961 263
- CN-A- 103 740 965
- CN-A- 104 107 097
- CN-A- 104 357 700
- CN-A- 104 357 700
- US-A1- 2013 011 691
- US-A1- 2014 348 688
- US-B1- 6 461 562

## Description

### TECHNICAL FIELD

The present invention relates to a porous metal material, particularly to a porous metal material with multilevel porous structure and the preparation method thereof.

### BACKGROUND

The porous metal material is used as a new engineering material which has functional and structural characteristics, and has an excellent property. In fields of metallurgical machinery, petrochemical engineering, energy sources, environmental protection, national defense and military industry, nuclear technology, biological pharmacy, medical equipment, etc., porous metal material has been widely used. Particularly, the porous metal material has been widely used in the medical care industry, and especially used as an implant. Currently, the most common medical porous metal materials are the materials with monotonous pore openings. Compared to the original dense material, the overall elasticity modulus of such kind of material is significantly reduced. It is reported in literature that the elasticity modulus of prepared porous titanium is 0.1 GPa-30 GPa. The elasticity modulus of porous tantalum is 1.53 GPa-7.0 GPa. The elasticity modulus of porous niobium is 0.14 GPa-1.08GPa. The elasticity modulus of porous tantalum niobium alloy is 1.6 GPa-7.0 GPa. The elasticity modulus of porous Ni-Ti shape memory alloy is 6 GPa-18 GPa. However, since the cavity walls of the pore cavity of such type of porous metal material are dense material, the elasticity modulus of the cavity wall itself is not actually reduced.

In the recent decade, a new material in the filed porous metal materials multilevel porous metal material has become an international research hotspot due to its unique properties. The multilevel porous metal material can be used in various fields like catalysis, separation, energy sources, optics, biological technology, bio-pharmaceutical, etc. Researchers not only research its material structure, but also pay attention to the preparation method thereof. Meanwhile, people's demand for multilevel porous metal material has further increased, especially multilevel porous metal material for medical use. It is necessary to redesign many aspects including the pore structure, the pore connectivity, physical and chemical properties of the material such as the elasticity modulus, etc., so as to meet different requirements. However, regarding existing matured multilevel porous metal material, since the pore structure is not designed reasonably, respective property index is not definite enough, and the prepared multilevel porous metal material cannot fully meet application requirements. Moreover, it is difficult for the preparation method of the above multilevel porous metal material to produce the multilevel porous metal material with structural characteristics that meet actual requirements and have controllable properties, particularly multilevel porous metal material with high strength and good toughness.

CN104107097A discloses a bone restoration with a macroscopic-microcosmic-nanometer hierarchy structure and mechanical adaptation and the preparation method thereof. A bone restoration is introduced, including macroscopic pore opening metal structure body, microscopic pore opening structure body, and nanofiber. The size of inner macroscopic pore opening is 300-1500 micrometer. Respective macroscopic pore openings are completely and mutually interconnected. Microscopic pore opening structure body is located inside the pore of macroscopic pore opening metal structure body. Inner microscopic pore opening structure is uniform, and pore openings are completely and mutually interconnected. The size of pore opening is 50-250 micrometer. The pore opening wall of microscopic pore opening is formed by nanofiber, the preparation method thereof is as follows. Firstly, macroscopic pore opening metal structure body is prepared by 3D print technology. Next, biodegradable polymer material is made into solution using an organic solvent. The solution is poured into the macroscopic pore opening of the macroscopic pore opening metal structure body. Next, a freezing treatment is performed. Next, thermally induced phase separation is performed. The elasticity modulus of the macroscopic pore opening metal structure body is 0.5-30GPa. The prepared bone restoration only has two levels of pores. Microscopic pore opening structure body is only located on the surface of the cavity wall of the macroscopic pore opening, rather than inside. The cavity wall of the microscopic pore opening structure body itself is a dense entity. Thus, although microscopic pore openings are completely and mutually interconnected, the capacity of transmitting the liquid is limited. Because of the addition and the randomness of the microscopic pore opening structure body, the overall elasticity modulus of the finally-obtained material is not definite. Also, the elasticity modulus of microscopic pore opening structure body is not definite. Moreover, the uniformity of the thickness of the microscopic pore opening structure body on the surface of the cavity wall of the macroscopic pore opening cannot be definite. Also, it is difficult to ensure the uniformity of its properties. Thus, it is difficult to meet the functional requirement of artificial bone.

The following patent/patent application documents relate to porous metal material: US 2013/001691, is to provide a porous tantalum used for medical implantation with good biocompatibility and biosafety, aims to solve the problem that the existing porous tantalum for medical implantation does not have well distributed interconnecting pores. US 2014/348688, is to provide a fabrication method for a biocompatible porous titanium construct.

### SUMMARY OF THE INVENTION

The purpose of the present invention is to provide a porous metal material, which is particularly suitable to be used as medical implant material and can make the cell sense the stress stimulation, eliminating cell stress shielding.

Another purpose of the present invention is to provide a preparation method of the porous metal material. The preparation method makes the structure of the porous metal material controllable, simple and easy.

Though researchers have already explored some new porous metal materials with multilevel porous structure, the current situation is that the connectivity of the porous metal material is insufficient. Macropores and micropores cannot be effectively controlled. Also, what kind of porous metal material with porous structure can meet the applicable requirements of a variety of functions? What kind of property index is the one truly needed by application scenario to achieve desired properties? For example, for medical regeneration material, what kind of porous metal material can ensure smooth and uniform transmission of the biological tissue solution? What kind of elasticity modulus can make biological tissue grow smoothly and normally? Researchers are expected to further explore and figure out answers to the above questions.

During the research, the inventor recognizes that after the porous material, used as medical material, is implanted into human body, cells will adhere to the cavity wall of the pore cavity. If the cavity wall is a dense entity, the elasticity modulus of the cavity wall is high. Thus, the cavity wall is not prone to deform together with the overall deformation. In that case, it will be difficult for cells to sense stress stimulation, generating stress shielding of cells. In the inventor's view, if the cavity wall of the pore cavity is provided with pore cavities, and the pore size of the pore cavity is also suitable, the elasticity modulus of the cavity wall of the pore cavity of the original dense entity can be reduced. Thus, cells adhered on the cavity wall of the pore cavity can sense the stress stimulation. Moreover, a method with which the above material can be effectively prepared is provided. Such type of material can eliminate cell stress shielding, fully and accurately meeting the functional requirement of medical porous material.

The purpose of the present invention is achieved through the porous metal material of claim 1, in more detail through the following technical solution:
A porous metal material is provided, comprising a material body. The material body is multilevel porous metal material. That is, the material body is formed by a plurality of levels of porous metal material. The multilevel porous metal material is classified based on the pore size of the material. The levels are classified at least more than two levels. In the multilevel porous metal material, a pore size of the smallest level of porous metal material is less than 1 micrometer, and the elasticity modulus of the smallest level of porous metal material is less than 80GPa, and the porosity is no less than 40%, wherein the material body is formed by respective level of pore cavities classified based on the pore sizes of the material and respective level of cavity walls surrounding to form the pore cavities; the cavity wall, surrounding a three-dimensional space to form upper level of pore cavities, is formed by lower level of porous metal materials; pore cavities of different levels are interconnected with each other, and pore cavities within each level are also interconnected with each other; wherein, when the number of classified levels of the multilevel porous metal material is three levels, a first level of pore cavities have micrometer level pores, a third level of pore cavities, being the smallest level, have nanometer level pores, a pore size of a second level of pore cavities is between the pore size of the first level of pore cavities and the pore size of the third level of pore cavities, wherein the porosity refers to the porosity of the material which only has this level of pores cavities. Since the porous metal material with such structure is classified based on the pore size of the material, and the elasticity modulus of the porous metal material in different levels is designed in such a way that when the elasticity modulus of the smallest level of porous metal material is less than 80GPa, the porosity is no less than 40%. Thus, the material is particularly suitable to be used as medical implant material. When the pore size of the smallest level of porous metal material in the multilevel porous metal material is less than 1 micrometer, for example, as for human body, the smallest cell - lymph cell, the size of which is 6µm, the pore size of the pore cavity of the smallest level of porous metal material is less than1µm, which is less than the size of lymph cell. When such material is used as implant material, lymph cell can adhere to the cavity wall of the pore cavity. When the cavity wall bears stress, since its elasticity modulus is less than 80GPa which is less than the elasticity modulus value of general medical metal material, the cavity wall is prone to deformation, and lymph cells can sense the deformation brought by the stress, such that the stress shielding of the growth of cells can be significantly eliminated.

Furthermore, the material body is formed by respective level of pore cavities that are classified based on the pore size of the material and respective level of cavity walls surrounding to form the pore cavities. The cavity wall surrounding three-dimensional space to form the upper level of pore cavities is formed by the lower level of porous material. Different level of pore cavities are interconnected with each other, and pore cavities within each level are also interconnected with each other. Porous metal material with such structure is used as medical implant material, the pore size of which is classified into levels, meeting various requirements of the growth of biological tissue, ensuring the smoothly and fully transmission of the tissue fluid and metabolite, facilitating the smooth growth of the tissue.

Furthermore, when the number of classified levels of the multilevel porous metal material are three, the first level of pore cavities of the largest level are micrometer level pores, and the third level of pore cavities of the smallest level are nanometer level pore, and the pore size of the second level of pore cavities of the middle level is between the pore size of the first level of pores cavities and the pore size of the third level of pores cavities. Such material is particularly suitable to be used as regeneration material for bio- logical bone tissue implants.

Furthermore, in the multilevel porous metal material, the elasticity modulus of the upper level of porous metal material, which has pores that are one level larger than the smallest level of pore cavities, is less than 60GPa. The porosity is no less than 48%. The porosity refers to the porosity of the material which only forms that level of pore cavities. The previous level of porous material formed by pore cavities having pores that are one level larger than the smallest level of pore cavities and cavity walls refers to the porous material which has a single level of pore cavities, which still is an independent level of porous material in the multilevel porous material, and the pore cavities of which are one level larger than the size of the smallest level of pore cavities.

Furthermore, in the multilevel porous metal material, the elasticity modulus of the upper level of the porous metal material, having pores that are two levels larger than the smallest level of pore cavities, is less than 30GPa. The porosity is no less than 63%. The porosity refers to the porosity of the material which forms that level of pore cavities. Similarly, upper porous metal material which is two levels larger than the smallest level of porous metal material, also refers to the porous metal material which has such pore cavities, is also an independent level of porous metal material in the multilevel porous metal material, and the pore cavities of which are two levels larger than the size of the smallest level of pore cavities.

Furthermore, the porous metal material in within each level of the material body is a continuous structure body, such that each level of the porous metal material can be used as an independent level of porous metal material, existing in the body, and playing a special role of the level of pores in particular application scenario.

Furthermore, the maximum outer boundary of the continuous structure body formed by the porous metal material in the same level is equivalent to the maximum space boundary of the entire material body, such that each level of porous metal material can be used as an independent level of porous metal material, existing in the material body, and playing a special role of the level of pores. The porous metal material in the same level has its own unique physical and chemical properties such as elasticity modulus and so on. Thus, it can better meet various functional requirements in applicable scenarios.

Furthermore, pore cavities of the porous metal material in each level of the multi-level porous metal material are uniformly distributed and filled within the material body, such that the properties of this level of porous metal material and the properties of overall multilevel porous metal material are uniform and stable.

Furthermore, the porous metal material is particularly suitable to be used as medical implant regeneration material, that is, the material can induce the tissue regeneration and is used as bioactive replacement material to regenerate and repair pathological or coloboma tissue.

Furthermore, the metal is one or more items selected from the group consisting of tantalum, niobium, tantalum niobium alloy, medical titanium-based alloy, medical stainless steel, medical cobalt-based alloy.

Another purpose of the present invention is defined by claim 7; in more detail it is achieved as below:
A preparation method of the porous metal material, including the following steps:
(1) Material Preparation
   The raw material powder used to prepare porous metal material and the pore-forming agent used to prepare the pore cavities of the smallest level of the porous metal material of the multilevel porous metal material are mixed to prepare the slurry.
   The slurry is uniformly filled into polymer material support, so as to form a green body. The green body is crushed and dried, so as to obtain mixed grains containing the raw material powder, the pore-forming agent, and the polymer support material.
(2) The above obtained mixed grains and the pore-forming agent used to prepare pore cavities of the upper level of porous metal material that are larger than the pore cavities of the smallest level of porous metal material of the multilevel porous metal material are uniformly mixed to prepare the compact green body.
(3) The compact green body is sintered in vacuum. The sintered green body is sub-j ected to conventional follow-up treatment based on the treatment process of raw material used to prepare the porous metal material, so as to obtain the porous metal material.

After the above compact green body is sintered in vacuum, two kinds of pore-forming agent material evaporate, so as to form two levels of pore cavities, such that multilevel porous metal material of which the number of level and the size of pore cavities are controllable can be prepared. The crushed polymer material support material evaporates, enhancing the connectivity of the metal material.

Further, during the preparation of the above porous metal material, before preparing the compact green body, firstly, mixed grains are uniformly mixed with the pore-forming agent used to prepare the pore cavities which are one level larger than the pore cavities of the smallest level of porous metal material of the multilevel porous metal material. The mixture is uniformly poured into the polymer material support. The pore size value of the pore cavity of the polymer material support is more than a large value in the particle size of mixed grains and the particle size of the pore-forming agent. The strut of the polymer material support is used as a pore-forming agent used to prepare the pore cavities which are two levels larger than the smallest level of pore cavities of the multi- level porous metal material. As such, after the sintering in vacuum, the multilevel porous material which has three levels of pores can be prepared. Similarly, porous material with more levels of pores can be prepared.

Further, in the above preparation method of the porous metal material, the pore cavities of the polymer material support in which the pore size of the pore cavities is larger than the particle size of mixed grains and the pore size of the pore-forming agent, are three-dimensional interconnected, such that three-dimensional interconnected multilevel porous metal material can be prepared.

Advantages of the present invention are as below:
(1) In the porous metal material with the multilevel porous structure provided by the present invention, multiple levels of pores are designed to have the structure with levels classified based on the pore size of the material, facilitating regeneration of biological tissues. The pore size of the pore cavities of the smallest level of porous metal material is less than 1 micrometer, which is less than the size of biological cells. Moreover, when the elasticity modulus of the smallest level of porous metal material is less than 80GPa, and the porosity is no less than 40%, even the smallest cell adhered to the cavity walls of the pore cavities can sense the deformation, bearing the stress stimulation. Thus, the stress shielding of the growth of cells can be eliminated. Such material is particularly suitable to be used as medical implant material. For example, regarding the human body, the smallest cell - lymph cell has a size of 6µm. If the pore size of the smallest level of porous metal material is less than 1µm, during the growth of the lymph cell, corresponding stress shielding can be eliminated, such that the lymph cell can sense the stress stimulation. Thus, the cell growth can be facilitated. Such material becomes truly medical regeneration material, rather than the medical implant material which is only used as the support.
(2) In the porous metal material provided by the present invention, the material body is formed by the pore cavities classified based on the pore size of the material and cavity wall surrounding to form the pore cavities. The cavity wall surrounding three-dimensional space to form the pore cavities of the upper level of porous metal material is formed by the lower level of porous metal material. Pore cavities of different level of porous metal material respectively are interconnected with each other. Pore cavities within each level of porous material are also interconnected with each other. Porous metal material with such structure ensures the material to achieve three-dimensional interconnection within entire material body. The connectivity is good, ensuring the smooth and complete transmission of the tissue fluid and metabolite, facilitating the smooth growth of the tissue. The porous metal material with such structure can make overall elasticity modulus of the material body significantly reduced compared to the elasticity modulus of the original material itself. Compared to the porous metal material with a single type of pore openings, the elasticity modulus is further reduced. Moreover, each level of porous material in the same level within the material body is a continuous structure. The maximum outer boundary of each level of porous material in the same level is substantially equivalent to the space boundary of the entire material body. Thus, the porous metal material which has respective elasticity modulus value under different scales can be achieved. When the multilevel pores have the structure with three levels of pores, the first level of pore cavities are micrometer level pores, and the third level of pore cavities, being the smallest cavity, are nanometer level pores, and the pore size of the second level of pore cavities is between the pore size of the first level of pore cavities and the pore size of the third level of pore cavities. The material is particularly suitable to be used as regeneration material for biological bone tissue implanting. The first level of large pore cavities makes overall elasticity modulus of the material reduced significantly compared to the elasticity modulus of the dense material. Also, the first level of large pore cavities are used for tissues and vessels to grow. The second level of pore cavities are used for cells to stay. The elasticity modulus of the second level of pore cavities can make tissues and vessels within the first level of large pore cavities sense the impact of force. The elasticity modulus of the third level of pore cavities can make cells staying in the cavity wall of the second level of pore cavities sense the stress to facilitate cell division, creating conditions for cell division, facilitating cell division and growth. Thus, the material is a true bone tissue regeneration material suitable for medical implants.
(3) In the porous metal material provided by the present invention, since respective level of pore cavities are uniformly distributed within the material body, the properties of respective level of porous material within the material body are uniform and stable.
(4) The present invention provides the preparation method of porous metal mate- rial, with which multilevel porous structure can be prepared. The metal raw material powder is mixed with the pore-forming agent used to prepare the smallest level of pore cavities, so as to prepare the slurry. The slurry is uniformly filled into polymer material support to form a green body. The green body is dried and crushed to obtain mixed grains containing the raw material powder, the pore-forming agent and the polymer material support material. Mixed grains are uniformly mixed with the pore-forming agent used to prepare the upper level of pore cavities that are larger than the smallest level of pore cavities to prepare compact green body, such that the cavity wall of the upper level of pore cavities which is formed by the lower level of porous material can be achieved. Moreover, each level of porous material within the material body can be ensured to be a continuous structure. Also, the maximum outer boundary of each level of porous material in the same level which is substantially equivalent to the space boundary of the entire material body can be achieved. Thus, each level of porous material has its unique physical and chemical properties. Furthermore, the size of the pore cavity, the connectivity between pore cavities, and the elasticity modulus value of respective level of pore material can be effectively controlled. The method is simple and easy to achieve. Parameters are easy to adjust and control.

### BRIEF DESCRIPTION OF THE DRAWINGS

Hereinafter, the present invention will be further described with reference to the accompanying drawings and embodiments.
FIG. 1 is a schematic diagram of the porous material of the present invention; 1-1 is the front view, 1-2 is the left view, 1-3 is the top view;
FIG. 2 is an enlarged view of portion A in FIG. 1;
FIG. 3 is a cross-sectional view taken along B-B in FIG. 2.

### DETAILED DESCRIPTION OF THE INVENTION

Hereinafter, embodiments of the present invention are described with reference to drawings. Embodiments are provided based on the technical solution of the present invention. Detailed embodiments and specific operating procedure are provided. However, the protective scope of the present invention is not only limited to the following embodiments.

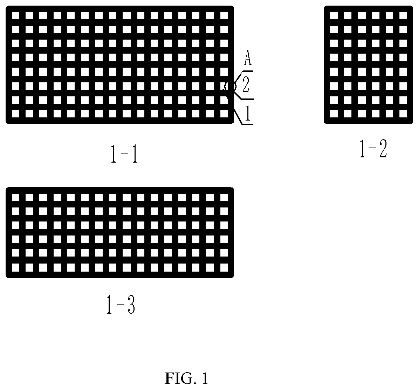

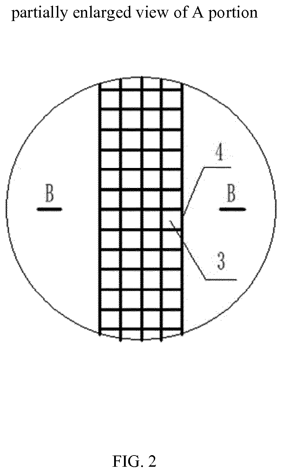

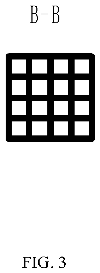

As shown in FIG. 1, the figure is a porous metal material with three-dimensional interconnected pores, wherein 1 is pore cavity, and 2 is the cavity wall of the pore cavity. It can be seen from FIG. 2, cavity wall 2 of pore cavity 1 is formed by smaller pore cavities 3 (the next level of pores) and cavity wall 4 surrounding the next level of pore cavities 3. With reference to FIG. 2 which is an enlarged view of cavity wall 2, and FIG. 3 which is a cross-sectional view taken along B-B, it can be seen that pore cavities 3 are three-dimensionally interconnected, and two levels of pores are also mutually three-dimensionally interconnected.

Similarly, porous material with multilevel porous structure can be formed with more than three levels.

Each level of porous material containing pore cavities 1 and pore cavities 3 within the material body is a continuous structure body.

Each level of porous material containing pore cavities 1 and pore cavities 3 thoroughly occupies the entire material body.

Respective level of pore cavities including pore cavity 1 and pore cavity 3 are uniformly distributed within the material body.

Hereinafter, embodiments of the present invention are provided in detail:

### Embodiment 1:

A porous metal material is provided. Metal tantalum powder is selected as the raw material of such material, including material body, wherein the material body is multilevel porous material. The multilevel porous material is classified based on the pore size of the material. The number of classified levels is three. The pore size of the first level of pore cavities is 400µm-600µm. The pore size of the second level of pore cavities is 25µm- 60µm. The pore size of the third level, being the smallest level, of pore cavities is 200nm- 500nm.

The material body of such porous material is formed by the pore cavity which is classified based on the pore size of the material and cavity wall surrounding to form the pore cavity. The cavity wall surrounding in the three-dimensional space to form the pore cavity of the previous level of porous material is formed by the next level of porous material. Pore cavities of each level of porous material respectively are interconnected with each other, and pore cavities of respective level of porous material are also interconnected with each other. Each level of porous material is a continuous structure body. The maximum outer boundary of each level of porous material is substantially equivalent to the space boundary of the entire material body. Pore cavities in the same level of porous material in each level of the multi-level porous material are distributed uniformly within the material body.

The preparation method thereof is as below:
(1) Material Preparation
   Tantalum powder with the particle size of 1µm-2µm is used as raw material. Urea with the particle size of 300nm-600nm is used as the pore-forming agent of the smallest level of pores. Polystyrene with the particle size of 300nm-600nm is used as a binder. The slurry is prepared based on a volume ratio of 1: 2: 1: 8 of tantalum powder: uea: polystyrene: distilled water.
   Polyester foam with the pore size of 100µm-200µm is used. The slurry is uniformly filled into the polyester foam with the foam impregnation method, so as to form a green body. The green body is dried and crushed to obtain mixed grains with the particle size of 30µm-70µm, containing raw material, pore-forming agent, and polyester foam.
(2) After the mixed grains are uniformly mixed, ethyl cellulose with the particle size of 30µm-70µm based on a volume ratio of 1: 2, the mixture is uniformly poured into three-dimensional interconnected polyester foam with a strut diameter of 500µm- 700µm and a pore size of 400µm-600µm. Next, the polyester foam is disposed into a closed mold to be pressed into the compact green body.
(3) The compact green body is sintered in vacuum. The sintered green body is sub-j ected to conventional follow-up heat treatment based on tantalum material processing, so as to obtain porous tantalum with a level number of three.

The cross-sectional direct observation method is used to test the porosity. The result is that the porosity of the first level of pores is 79%. The porosity refers to a porosity of the material which only has the first level of pore cavities. That is, during the calculation, the second and the third level of pore cavities are not taken into account (the second and the third level of pore cavities are deemed as a dense entity). The porosity of the third level of pore cavities is 64%. The porosity refers to a porosity of material which only has the third level of pore cavities. That is, during the calculation, analysis and calculation are performed on the material portion which only has the third level of pore cavities. The porosity of the second level of pores is 71%. The porosity refers to a porosity of material which only has the second level of pore cavities. That is, during the calculation, analysis and calculation are performed on the material portion which only has the second level and the third level of pore cavities. However, the third level of pore cavities are not taken into account. The material portion is deemed as a dense entity.

The nano-indentation method is used to measure the elasticity modulus of the second level of porous material and the third level of porous material. The elasticity modulus of porous tantalum with the second level of pore cavities is measured as 46GP. The elasticity modulus of porous tantalum with the third level of pore cavities is 71GP. Test instrument and parameters are as follows. G200 nano-indentation instrument is used. Berkovich type of pressure head made of equilateral triangle diamond is used as the pressure head.

Continuous stiffness measurement technology is used. During the test, the loading is performed at a loading rate of 0.005s⁻¹ to a predetermined maximum depth of 2000nm. The maximum load maintains for 100s. Next, unloading is performed at the same rate as that of loading, till 10% of the maximum load is left. The load maintains for 50s, till the unloading is finished completely. The status maintains for 10s to the end. The experiment temperature is 20 . 5 spots are tested. An average value is obtained.

Conventional foam impregnation method is used to prepare porous tantalum which only has the first level of pore cavities. Instron mechanics test machine is used to test compression stress-strain curve of the above porous tantalum sample at 25. Initial deformation shown in the stress-strain curve is elastic deformation. Elasticity modulus is taken as the ratio of stress value of the elastically deformed portion to corresponding strain value. The measured elasticity modulus is 1.9GPa.

Similarly, with the above testing method, the overall elasticity modulus of porous tantalum which has three levels of pore cavity structure is measured as 1.6GPa.

Such porous tantalum with three levels can be used as a bone regeneration material.

Animal implant experiment is performed on porous tantalum of the present embodiment and traditional porous tantalum product prepared by chemical vapor deposition (hereinafter, referred as traditional porous tantalum in short). The procedure and analysis are as follows:

### (1) Implant Material Preparation

The porous tantalum with the structure of three levels of pores prepared by the present embodiment is made into a sample with the size of 12mm×12mm×6mm. Traditional chemical vapor deposited porous tantalum is also made into a sample with the size of 12mm×12mm×6mm. The ultrasonic cleaning is conducted on implanting piece samples with distilled water, acetone solution, and 70% ethyl alcohol sequentially for 20min. Again, ultrasonic cleaning is conducted with distilled water for 15min. Then, high-pressure steam sterilization is performed.

### (2) Experiment Animal Preparation

9 healthy dogs of either gender are selected, with the weight of 10-13 kg. The dogs are randomly divided into a 4-week group, an 8-week group, and a 12-week group, with 3 dogs in each group.

### (3) Operation Implant Material

Pentobarbital is selected as the anesthetic, the total amount of which is calculated based on 30mg/Kg weight. The solution with a concentration of 1% is prepared with 0.9% normal saline. The solution is injected slowly via the ear vein to achieve anesthesia. After general anesthesia, the dog is fixed on the operation table. The skin and subcutaneous tissue on the inner side of left thighbone are cut open. The blunt separation is performed along muscle space to reach the thighbone. The periosteum is cut open, so as to expose thighbone cortex. A drilling machine is used to create a bone loss of 12mm×12mm×6mm. One porous tantalum sample prepared by the present embodiment is disposed inside. In the same way, one traditional porous tantalum sample is implanted in the right thighbone. The periosteum is sutured. The wound is sutured layer by layer. After the operation, 1.0g cephazolin sodium is injected intramuscularly for 3 days. After 10 days, stitches are taken out (for all 3 groups of dogs, 9 dogs in total). Their activities are not restricted.

An intravenous administration is conducted with 3mg/kg of sodium fluorescein and 90mg/kg of xylenol orange, so as to conduct fluorescein label.

### (4) analysis of the testing results

In 4, 8, and 12 weeks after the operation, the groups of dogs are executed respectively. The thighbone is taken out. After a treatment with 80% ethyl alcohol, the implant material is subject to dehydration, resin embedding, and hard tissue section. Each implanting piece is sliced into two sections, wherein one section is dyed with toluidine blue or HE.

The hard tissue section is observed under fluorescence microscope. Under the fluorescence microscope, xylenol orange emits orange light, while sodium fluorescein emits green light.

In 4 weeks after the implantation, fluorescence strip is mainly located in the host bone surface near the implanted part and is in a linear parallel distribution. In a direction from the host bone surface to the implanted part, orange fluorescence and green fluorescence occur sequentially. The difference between two kinds of implanting pieces is not obvious.

In 8 weeks after the implantation, in two kinds of implanting pieces, fluorescence strips have already contacted the surface of the implanting pieces and begun to extend into pore openings. The orange fluorescence is in a piece-like and bulk-like distribution. The green fluorescence is in a linear distribution, extending into pore openings. Fluorescence extending into pore openings of the porous tantalum prepared by the present embodiment is more than that of traditional porous tantalum.

In 12 weeks after the implantation, in pore openings of implanting piece, a great amount of orange and green fluorescence can be seen. The distribution does not have a certain rule. Fluorescence strips are intertwined and overlapped with each other. Traditional porous tantalum fluorescein only deposits in pore openings near the surface of the implanted part. There is no fluorescence in deep pore openings. Deep pore openings of the porous tantalum prepared by the present embodiment have a great amount of fluorescein deposition.

Hard tissue section is dyed with toluidine blue or HE to be observed. Under the optical microscope, osteoblast is orange, osteoid is amaranth, newly mineralized bone is blue, and matured bone is green.

In 4 weeks after the implantation, gaps exist between two kinds of implanting pieces and host bone. Fibrous connective tissue can be seen in gaps and has a light orange color. Bone surface is amaranth and is undifferentiated and immatures osteoid.

In 8 weeks after the implantation, gaps between two kinds of implanting pieces and host bone are reduced. The regenerated bone tissue has contacted the surface of the implanted part, and begun to grow into pore openings. In the pore openings of surface of implanting piece and the pore openings near the surface, non-mineralized osteoid can be seen. The inner side of the pore opening in the deep portion of the implanting piece includes fibrous tissue. The regenerated bone tissue and fibrous tissue has grown into pore openings of porous tantalum implant piece prepared by the embodiment are more than those of traditional porous tantalum.

In 12 weeks after the implantation, surfaces of two kinds of implanting pieces have formed synostosis with bone tissue. Moreover, the bone tissue inside the pore opening has differentiated, matured and mineralized. In traditional porous tantalum implanting piece, bone tissue has only grown into superficial pore openings of the implanting piece. In deep pore openings of implanting piece, only a small amount of osteoid and fibrous tissue can be seen. In the porous tantalum implant piece prepared by the embodiment, calcified and matured bone tissue can also be seen in deep pore openings of the implant piece, with blood capillaries passing therethrough.

Further, the hard tissue section is observed under a low-magnification optical microscope. The bone growing depth is measured with image processing system. Results show that the bone growth of the porous tantalum implanting piece prepared by the embodiment is 32% more than that of traditional porous tantalum.

Experimental and analysis results show that porous tantalum with the structure of three levels of pores prepared by the present embodiment is pretty suitable to be used as bone repair material. The overall elasticity modulus and the value of the elasticity modulus of the smallest level facilitate the bone tissue and cells to sense the stress stimulation, promoting the growth of the bone tissue and cells. The first level of large pore cavities makes overall elasticity modulus of the material reduced significantly, compared to the elasticity modulus of the dense material, eliminating the bone tissue stress shielding, and facilitating the growth of tissue and vessels. The second level of pore cavities is used for cells to stay. The elasticity modulus of the third level of pore cavities enables cells staying in the cavity wall of the second level of pore cavities to sense the stress, facilitating cell division, eliminating the cell stress shielding, creating conditions for cell division, facilitating cell division and growth. Thus, it is truly suitable for medical implanting bone tissue repair and regeneration material.

### Embodiment 2:

A porous niobium material, which is multilevel porous material, is classified based on the pore size of the material. The number of classified levels is three levels. The pore size of the first level of pores cavities is 800µm-1500µm. The pore size of the second level of pores cavities is 20µm-60µm. The pore size of the third level, being the smallest level, of pores cavities is 100nm- 350nm.

The material body of such porous material is formed by the pore cavities which are classified based on the pore size of the material and cavity wall surrounding to form the pore cavities. The cavity wall surrounding in the three-dimensional space to form the pore cavity of the upper level of porous material is formed by the lower level of porous material. Pore cavities of different level of porous material respectively are interconnected with each other, and pore cavities within respective level of porous material are also interconnected with each other. Each level of porous material is a continuous structure. The maximum outer boundary of each level of porous material is substantially equivalent to the space boundary of the entire material body. Pore cavities in the same level of porous material are distributed uniformly within the material body.

The preparation method thereof is as below:
(1) Material Preparation
   Niobium powder with the particle size of 1µm-2µm is used as the raw material. Methyl cellulose with the particle size of 200nm-450nm is used as the pore pore-forming agent of the smallest level of pores. Polystyrene with the particle size of 200nm-450nm is used as the binder. The slurry is prepared based on a volume ratio of 1: 1.5: 1: 7.5 based on niobium powder: methyl cellulose: polystyrene: distilled water.
   Polyester foam with the pore size of 100µm-200µm is used. The slurry is uniformly filled into the polyester foam with the foam impregnation method, so as to form a green body. The green body is dried and crushed to obtain mixed grains with the particle size of 25µm-70µm, containing raw material, pore-forming agent, and polyester foam.
(2) After the mixed grains are uniformly mixed with ethyl cellulose with the particle size of 25µm-70µm based on a volume ratio of 1: 2, the mixture is uniformly poured into three-dimensional interconnected polyester foam with the strut diameter of 900µm-1600µm and the pore size of 400µm-600µm. Next, the polyester foam is disposed into a closed mold to be pressed into the compact green body.
(3) The compact green body is sintered in vacuum. The sintered green body is sub-j ected to conventional follow-up heat treatment based on niobium material processing to obtain porous niobium with a level number of three. Based on the testing method and the preparation method of Embodiment 1. The porosity of the first level of pores of such porous niobium is tested as 78%. The elasticity modulus is 0.8GPa. The porosity of the second level of pores is 48%. The elasticity modulus is 60 GPa. The porosity of the third level of pores is 40%. The elasticity modulus is 79 GPa. The overall elasticity modulus is 0.65GPa.

Such porous niobium with three levels can be used as a bone regeneration material.

### Embodiment 3 (comparative example)

A porous titanium material, which is multilevel porous material, is classified based on the pore size of the material. The number of classified levels are two, wherein the pore size of the pore cavity of the small pore is 250nm-470nm. The pore size of the pore cavity of large pore is 130µm-360µm.

The material body of such porous material is formed by the pore cavity which is classified based on the pore size of the material and cavity wall surrounding to form the pore cavity. The cavity wall surrounding in the three-dimensional space to form the pore cavity of the upper level of porous material is formed by the lower level of porous material. Pore cavities of each level of porous material respectively are interconnected with each other, and pore cavities of respective level of porous material are also interconnected with each other.

The preparation method thereof is as below:
(1) Material Preparation
   Titanium powder with the size of 1µm-3µm is used. Ammonium chloride with the particle size of 350nm-570nm is used as the pore pore-forming agent of the smallest level of pores. Titanium powder is uniformly mixed with ammonium chloride. Starch with the size of 350nm-570nm is used as the binder. The slurry is prepared based on a volume ratio of 1: 1.5: 1: 8 of titanium powder: ammonium chloride: starch: distilled water.
   The slurry is uniformly filled into the polyester foam with a strut diameter of 200µm-450µm with the foam impregnation method, so as to form a green body. The green body is dried and crushed to obtain mixed grains with the particle size of 200µm-450µm, containing titanium powder, pore-forming agent, and polyester foam.
(2) After the mixed grains are uniformly mixed with methyl cellulose with the particle size of 200µm-450µm based on a volume ratio of 1: 3, the mixture is disposed into a closed mold to be pressed into the compact green body.
(3) The compact green body is sintered in vacuum. The sintered green body is subjected to follow-up processing based on the conventional processing of titanium to obtain porous titanium with the level number of two.
Based on the testing method and the preparation method of Embodiment 1, the porosity of the first level of pores of such porous titanium is tested as 63%. The elasticity modulus is 30GPa. The porosity of the second level of pores is 40%. The elasticity modulus is 80 GPa. The overall elasticity modulus is 27GPa.

Such porous titanium with two levels can be used as a bone implant material.

### Embodiment 4:

A porous material is provided. Metal 316L stainless steel alloy powder is selected as the raw material powder of the material, includes material body, wherein the material body is multilevel porous material, the multilevel porous material is classified based on the pore size of the material. The number of classified levels are three levels. The pore size of the first level of pore cavities is 200µm-400µm. The pore size of the second level of pore cavities is 40µm-80µm. The pore size of the third level, being the smallest level, of pore cavities is 300nm-600nm.

The material body of such porous material is formed by the pore cavity which is classified based on the pore size of the material and cavity wall surrounding to form the pore cavity. The cavity wall surrounding in the three-dimensional space to form the pore cavity of the upper level of porous material is formed by the lower level of porous material. Pore cavities of different levels of porous material respectively are interconnected with each other, and pore cavities within each level of porous material are also interconnected with each other. Each level of porous material is a continuous structure body. Pore cavities in the same level of porous material in the multilevel porous material are distributed uniformly within the material body.

The preparation method thereof is as below:
(1) Material Preparation
   316L stainless steel powder with the particle size of 1µm-3µm is used as the raw material. Starch with the particle size of 400nm-700nm is used as the pore pore-forming agent of the smallest level of pores. Stearic acid with the particle size of400nm-700nm is used as the binder. The slurry is prepared based on a volume ratio of 1: 2: 1: 9 of 316L stainless steel powder: starch: stearic acid: distilled water.
   Polyester foam with the pore size of 400µm-700µm is used. The slurry is uniformly filled into the polyester foam with the foam impregnation method, so as to form a green body. The green body is dried and crushed to obtain mixed grains with the particle size of 50µm-90µm, containing raw material, pore-forming agent, and polyester foam.
(2) The mixed grains are uniformly mixed with ammonium sulfate with the particle size of 50µm-90µm based on a volume ratio of 1: 2. The mixture is uniformly poured into three-dimensional interconnected polyester foam with the strut diameter of 300µm-500µm and the pore size of 300µm-500µm. Next, the polyester foam is disposed into a closed mold to be pressed into the compact green body.
(3) The compact green body is sintered in vacuum. The sintered green body is subjected to conventional follow-up heat treatment based on the processing of 316L stainless steel material to obtain porous 316L stainless steel with three levels.

Based on the testing method and the preparation method of Embodiment 1, the porosity of the first level of pores of such porous 316L stainless steel is tested as 79%. The elasticity modulus is 26GPa. The porosity of the second level of pores is 70%. The elasticity modulus is 54GPa. The porosity of the third level of pores is 65%. The elasticity modulus is 75 GPa. The overall elasticity modulus is 21 GPa.

Such porous 316L stainless steel with a level number of three can be used as a bone regeneration material.

### Embodiment 5:

A porous material is provided. Metal alloy CoNiCrMo (F562) is selected as the raw material of the material. The material includes a material body, wherein the material body is multilevel porous material. The multilevel porous material is classified based on the pore size of the material. The number of classified levels are three levels. The pore size of the first level of pore cavities is 350µm-560µm. The pore size of the second level of pore cavities is 15µm-50µm. The pore size of the third level, being the smallest level, of pores cavities is 1m-45nm.

The material body of such porous material is formed by the pore cavities which are classified based on the pore size of the material and cavity wall surrounding to form the pore cavities. The cavity wall surrounding the three-dimensional space to form the pore cavity of the upper level of porous material is formed by the lower level of porous material. Pore cavities of different level of porous material respectively are interconnected with each other, and pore cavities within each level of porous material are also interconnected with each other. Each level of porous material is a continuous structure body. The maximum outer boundary of each level of porous material is substantially equivalent to the space boundary of the entire material body. Pore cavities in the same level of porous material in the multilevel porous material are distributed uniformly within the material body.

The preparation method thereof is as below:
(1) Material Preparation
   CoNiCrMo alloy powder with the particle size of 1µm-2µm is used as raw material. Urea with the particle size of 10nm-60nm is used as the pore pore-forming agent of the smallest level of pores. Polystyrene with the particle size of 10nm- 60nm is used as a binder. the slurry is prepared based on a volume ratio of 1: 1.5: 1: 8 of CoNiCrMo alloy powder: urea: polystyrene: distilled water.
   Polyester foam with a pore size of 350µm-700µm is used. The slurry is uniformly filled into the polyester foam with the foam impregnation method, so as to form a green body. The green body is dried and crushed to obtain mixed grains with the particle size of 20µm-60µm, containing raw material, pore-forming agent, and polyester foam.
(2) After mixed grains are uniformly mixed with ethyl cellulose with the particle size of 20µm-60µm based on a volume ratio of 1: 2, the mixture is uniformly poured into three- dimensional interconnected polyester foam with the pore size of 200µm-400µm and a strut diameter of 450µm-650µm. Next, the polyester foam is disposed into a closed mold to be pressed into the compact green body.
(3) The compact green body is sintered in vacuum. The sintered green body is subjected to conventional follow-up heat treatment based on CoNiCrMo alloy processing, so as to obtain porous CoNiCrMo alloy with a level number of three.

Based on the testing method and the preparation method of Embodiment 1, the porosity of the first level of pores of such porous CoNiCrMo alloy is tested as 78%. The elasticity modulus is 30GPa. The porosity of the second level of pores is 69%. The elasticity modulus is 60 GPa. The porosity of the third level of pores is 64%. The elasticity modulus is 80GPa. The overall elasticity modulus is 25 GPa.

Such porous CoNiCrMo alloy with a level number of three can be used as a bone regeneration material.

## Claims

1. A porous metal material, comprising
a material body;
**characterized in that**
the material body is a multilevel porous metal material;
the multilevel porous metal material is classified based on pore sizes of the material;
the levels are classified into at least more than two levels;
in the multilevel porous metal material, a pore size of a smallest level of porous metal material is less than 1 micrometer;
an elasticity modulus of the smallest level of porous metal material is less than 80GPa;
a porosity is no less than 40%;
wherein the material body is formed by respective level of pore cavities classified based on the pore sizes of the material and respective level of cavity walls surrounding to form the pore cavities;
the cavity wall, surrounding a three-dimensional space to form upper level of pore cavities, is formed by lower level of porous metal materials;
pore cavities of different levels are interconnected with each other, and
pore cavities within each level are also interconnected with each other;
**characterized in that**
when the number of classified levels of the multilevel porous metal material is three levels, a first level of pore cavities have micrometer level pores, a third level of pore cavities, being the smallest level, have nanometer level pores, a pore size of a second level of pore cavities is between the pore size of the first level of pore cavities and the pore size of the third level of pore cavities.

2. The porous metal material of claim 1, **characterized in that**
in the multilevel porous metal material, an elasticity modulus of an upper level of porous metal material having pores that are one level larger than the smallest level of pore cavities is less than 60GPa, and a porosity is no less than 48%.

3. The porous metal material any of claims 1-2, **characterized in that** in the multilevel porous metal material, an elasticity modulus of an upper level of porous metal material having pores that are two levels larger than the smallest level of pore cavities is less than 30GPa, and a porosity is no less than 63%.

4. The porous metal material of any of claims 1-3, **characterized in that** pore cavities of the porous metal material in the same level of the multilevel porous metal material are distributed uniformly within the material body.

5. The porous metal material of claim 1, **characterized in that** the metal is one or more items selected from the group consisting of tantalum, niobium, tantalum niobium alloy, medical titanium-based alloy, medical stainless steel, and medical cobalt-based alloy.

6. Use of the porous metal material of any of claims 1-5, as a medical implant regeneration material.

7. A preparation method of a porous metal material of claim 1, **characterized in that** the porous metal material is prepared by the following steps:
(1) material preparation by mixing raw material powder used to prepare the porous metal material with a pore- forming agent used to prepare a pore cavity of a smallest level of the porous metal material of a multilevel porous metal material, and preparing a slurry; filling the slurry uniformly into a polymer material support, to form a green body; drying the green body; and crushing the green body to obtain mixed grains containing the raw material powders, the pore-forming agent, and the polymer material support material;
(2) uniformly mixing the above obtained mixed grains with a pore-forming agent used to prepare a pore cavity of an upper level of porous metal material which is larger than the pore cavity of the smallest level of porous metal material of the multilevel porous metal material, and pressing the mixture into a compact green body;
(3) sintering the compact green body in vacuum; conducting conventional follow-up processing on the sintered green body based on treatment processing of raw material used to prepare the porous metal material, so as to obtain the porous metal material.

8. The preparation method of the porous metal material of claim 7, **characterized in that** before preparing the compact green body, firstly, uniformly mixing the mixed grains with a pore-forming agent used to prepare a pore cavity which is one level larger than the pore cavity of the smallest level of porous metal material of the multilevel porous metal material;
uniformly pouring a mixture of the mixed grains and the pore-forming agent into the polymer material support;
wherein
a pore size value of a pore cavity of the polymer material support is more than a large value in a particle size of the mixed grains and a particle size of the pore-forming agent;
a strut of the polymer material support is used as a pore-forming agent used to prepare a pore cavity which is two levels larger than of the smallest level of pore cavities of the multilevel porous metal material.

9. The preparation method of the porous metal material of claim 7 or 8, **characterized in that** pore cavities of the polymer material support are three-dimensional interconnected.

## Patentansprüche

1. Poröses Metallmaterial, umfassend
einen Materialkörper;
**dadurch gekennzeichnet, dass**
der Materialkörper ein mehrstufiges poröses Metallmaterial ist;
das mehrstufige poröse Metallmaterial anhand der Porengrößen des Materials klassifiziert wird;
die Stufen in mindestens mehr als zwei Stufen unterteilt sind;
in dem mehrstufigen porösen Metallmaterial eine Porengröße einer kleinsten Stufe des porösen Metallmaterials weniger als 1 Mikrometer beträgt;
ein Elastizitätsmodul der kleinsten Stufe des porösen Metallmaterials weniger als 80 GPa beträgt;
eine Porosität mindestens 40 % ist;
wobei der Materialkörper durch eine jeweilige Stufe von Porenhohlräumen gebildet wird, die auf der Grundlage der Porengrößen des Materials und der jeweiligen Stufe von Hohlraumwänden, die die Porenhohlräume umgeben, klassifiziert sind;
die Hohlraumwand, die einen dreidimensionalen Raum umgibt, um die obere Stufe der Porenhohlräume zu bilden, durch eine untere Stufe aus porösen Metallmaterialen gebildet wird;
Porenhohlräume verschiedener Stufen miteinander verbunden sind und
Porenhohlräume innerhalb jeder Stufe ebenfalls miteinander verbunden sind;
**dadurch gekennzeichnet, dass**,
wenn die Anzahl der klassifizierten Stufen des mehrstufigen porösen Metallmaterials drei Stufen ist, eine erste Stufe von Porenhohlräumen Poren im Mikrometerbereich aufweist, eine dritte Stufe von Porenhohlräumen, das die kleinste Stufe ist, Poren im Nanometerbereich aufweist, eine die Porengröße einer zweiten Stufe von Porenhohlräumen zwischen der Porengröße der ersten Stufe von Porenhohlräumen und der Porengröße der dritten Stufe von Porenhohlräumen liegt.

2. Poröses Metallmaterial nach Anspruch 1, **dadurch gekennzeichnet, dass**
in dem mehrstufigen porösen Metallmaterial ein Elastizitätsmodul einer oberen Stufe des porösen Metallmaterials mit Poren, die eine Stufe größer sind als die kleinste Stufe der Porenhohlräume, weniger als 60 GPa beträgt und eine Porosität nicht weniger als 48 % beträgt.

3. Poröses Metallmaterial nach einem der Ansprüche 1 bis 2, **dadurch gekennzeichnet, dass** in dem mehrstufigen porösen Metallmaterial ein Elastizitätsmodul einer oberen Stufe des porösen Metallmaterials mit Poren, die zwei Stufen größer sind als die kleinste Stufe der Porenhohlräume, weniger als 30 GPa beträgt und eine Porosität nicht weniger als 63 % beträgt.

4. Poröses Metallmaterial nach einem der Ansprüche 1 bis 3, **dadurch gekennzeichnet, dass** Porenhohlräume des porösen Metallmaterials in der gleichen Stufe des mehrstufigen porösen Metallmaterials gleichmäßig innerhalb des Materialkörpers verteilt sind.

5. Poröses Metallmaterial nach Anspruch 1, **dadurch gekennzeichnet, dass** das Metall eines oder mehrere Elemente ist/sind, die aus der Gruppe ausgewählt aus Tantal, Niob, Tantal-Niob-Legierung, medizinischer Titanbasislegierung, medizinischem Edelstahl und medizinischer Kobaltbasislegierung besteht.

6. Verwendung des porösen Metallmaterials nach einem der Ansprüche 1 bis 5 als Regenerationsmaterial für medizinische Implantate.

7. Herstellungsverfahren für ein poröses Metallmaterial nach Anspruch 1, **dadurch gekennzeichnet, dass** das poröse Metallmaterial durch die folgenden Schritte hergestellt wird:
(1) Materialherstellung durch Mischen von Rohmaterialpulver, das zur Herstellung des porösen Metallmaterials verwendet wird, mit einem Porenbildungsmittel, das zur Herstellung eines Porenhohlraums einer kleinsten Stufe des porösen Metallmaterials eines mehrstufigen porösen Metallmaterials verwendet wird, und Herstellen einer Aufschlämmung; gleichmäßiges Einfüllen der Aufschlämmung in einen Polymermaterialträger, um einen Grünkörper zu bilden; Trocknen des Grünkörpers; und Zerkleinern des Grünkörpers, um Mischkörner zu erhalten, die die Rohmaterialpulver, das Porenbildungsmittel und das Polymermaterialträgermaterial enthalten;
(2) gleichmäßiges Mischen der oben erhaltenen Mischkörner mit einem Porenbildungsmittel, das verwendet wird, um einen Porenhohlraum einer oberen Stufe des porösen Metallmaterials herzustellen, der größer ist als der Porenhohlraum der kleinsten Stufe des porösen Metallmaterials des mehrstufigen porösen Metallmaterials, und Pressen der Mischung zu einem kompakten Grünkörper;
(3) Sintern des kompakten Grünkörpers im Vakuum; Durchführen einer konventionellen Nachbearbeitung des gesinterten Grünkörpers auf der Grundlage der Bearbeitung des zur Herstellung des porösen Metallmaterials verwendeten Rohmaterials, um das poröse Metallmaterial zu erhalten.

8. Verfahren zur Herstellung des porösen Metallmaterials nach Anspruch 7, **dadurch gekennzeichnet, dass** vor der Herstellung des kompakten Grünkörpers zunächst die Mischkörner gleichmäßig mit einem Porenbildungsmittel gemischt werden, das zur Herstellung eines Porenhohlraums verwendet wird, der eine Stufe größer ist als der Porenhohlraum der kleinsten Stufe des porösen Metallmaterials des mehrstufigen porösen Metallmaterials;
gleichmäßiges Gießen einer Mischung aus den Mischkörnern und dem Porenbildungsmittel in den Polymermaterialträger;
wobei
ein Porengrößenwert eines Porenhohlraums des Polymermaterialträgers größer ist als ein großer Wert in einer Teilchengröße der Mischkörner und einer Teilchengröße des Porenbildungsmittel;
eine Strebe des Polymermaterialträgers als Porenbildungsmittel verwendet wird, das verwendet wird, um einen Porenhohlraum herzustellen, der zwei Stufen größer ist als die kleinste Stufe der Porenhohlräume des mehrstufigen porösen Metallmaterials.

9. Verfahren zur Herstellung des porösen Metallmaterials nach Anspruch 7 oder 8, **dadurch gekennzeichnet, dass** die Porenhohlräume des Polymermaterialträgers dreidimensional miteinander verbunden sind.

## Revendications

1. Matériau métallique poreux comprenant
un corps de matériau;
**caractérisé par le fait que**
le corps de matériau est un matériau métallique poreux à niveaux multiples;
le matériau métallique poreux à niveaux multiples est classé sur la base des tailles de pores du matériau;
les niveaux sont classés en au moins plus de deux niveaux;
dans le matériau métallique poreux à niveaux multiples, une taille de pore d'un niveau le plus petit de matériau métallique poreux est inférieure à 1 micromètre;
un module d'élasticité du plus petit niveau de matériau métallique poreux est inférieur à 80 GPa;
une porosité n'est pas inférieure à 40 %;
dans lequel le corps de matériau est formé par un niveau respectif de cavités de pores qui sont classées sur la base des tailles de pores du matériau et du niveau respectif de parois de cavité entourant les cavités de pores;
la paroi de cavité qui entoure un espace tridimensionnel pour former le niveau supérieur de cavités de pores est formée par un niveau inférieur de matériaux métalliques poreux;
des cavités de pores de différents niveaux sont reliées entre elles, et
des cavités de pores à l'intérieur de chaque niveau sont également reliées entre elles;
**caractérisé par le fait que**
lorsque le nombre de niveaux classés du matériau métallique poreux à niveaux multiples est de trois niveaux, un premier niveau de cavités de pores présente des pores de niveau micrométrique, un troisième niveau de cavités de pores, qui est le niveau le plus petit, présente des pores de niveau nanométrique, une taille de pore d'un deuxième niveau de cavités de pores est compris entre la taille de pore du premier niveau de cavités de pores et la taille de pore du troisième niveau de cavités de pores.

2. Matériau métallique poreux selon la revendication 1, **caractérisé par le fait que**
dans le matériau métallique poreux à niveaux multiples, un module d'élasticité d'un niveau supérieur de matériau métallique poreux ayant des pores qui sont d'un niveau plus grand que le plus petit niveau de cavités de pores est inférieur à 60 GPa, et une porosité n'est pas inférieure à 48 %.

3. Matériau métallique poreux selon l'une quelconque des revendications 1 à 2, **caractérisé par le fait que**, dans le matériau métallique poreux à niveaux multiples, un module d'élasticité d'un niveau supérieur de matériau métallique poreux ayant des pores qui sont de deux niveaux plus grands que le plus petit niveau de cavités de pores est inférieur à 30 GPa, et une porosité n'est pas inférieure à 63 %.

4. Matériau métallique poreux selon l'une quelconque des revendications 1 à 3, **caractérisé par le fait que** des cavités de pores du matériau métallique poreux au même niveau du matériau métallique poreux à niveaux multiples sont réparties uniformément dans le corps de matériau.

5. Matériau métallique poreux selon la revendication 1, **caractérisé par le fait que** le métal est/sont un ou plusieurs éléments choisis dans le groupe constitué par le tantale, le niobium, l'alliage de tantale et de niobium, l'alliage médical à base de titane, l'acier inoxydable médical et l'alliage médical à base de cobalt.

6. Utilisation du matériau métallique poreux selon l'une quelconque des revendications 1 à 5, en tant que matériau de régénération d'implant médical.

7. Procédé de préparation d'un matériau métallique poreux selon la revendication 1, **caractérisé par le fait que** le matériau métallique poreux est préparé par les étapes suivantes:
(1) préparation de matériau en mélangeant de la poudre de matière première utilisée pour préparer le matériau métallique poreux avec un agent porogène utilisé pour préparer une cavité de pore d'un plus petit niveau du matériau métallique poreux d'un matériau métallique poreux à niveaux multiples, et en préparant une suspension; en versant la suspension uniformément dans un support en matériau polymère, pour former un corps cru; en séchant le corps cru; et en broyant le corps cru pour obtenir des grains mélangés contenant les poudres de matière première, l'agent porogène et le matériau de support en matériau polymère,
(2) mélanger uniformément les grains mélangés obtenus ci-dessus avec un agent porogène utilisé pour préparer une cavité de pore d'un niveau supérieur de matériau métallique poreux qui est plus grande que la cavité de pore du plus petit niveau de matériau métallique poreux du matériau métallique poreux à niveaux multiples, et presser le mélange pour obtenir un corps cru compact;
(3) fritter sous vide le corps cru compact, effectuer un traitement de suivi conventionnel sur le corps cru fritté sur la base du traitement de la matière première utilisée pour préparer le matériau métallique poreux, de manière à obtenir le matériau métallique poreux.

8. Procédé de préparation du matériau métallique poreux selon la revendication 7, **caractérisé par le fait qu'**avant de préparer le corps cru compact, on mélange d'abord uniformément les grains mélangés avec un agent porogène utilisé pour préparer une cavité de pore qui est d'un niveau plus grande que la cavité de pore du plus petit niveau de matériau métallique poreux du matériau métallique poreux à niveaux multiples;
verser uniformément un mélange des grains mélangés et de l'agent porogène dans le support en matériau polymère;
dans lequel
une valeur de taille de pore d'une cavité de pore du support en matériau polymère est supérieure à une grande valeur dans une taille de particule des grains mélangés et une taille de particule de l'agent porogène;
une entretoise du support en matériau polymère est utilisée en tant qu'agent porogène utilisé pour préparer une cavité de pore qui est de deux niveaux plus grande que le plus petit niveau de cavités de pore du matériau métallique poreux à niveaux multiples.

9. Procédé de préparation du matériau métallique poreux selon la revendication 7 ou 8, **caractérisé par le fait que** les cavités de pores du support en matériau polymère sont reliées entre elles en trois dimensions.
